# DEMANDE DE BREVET EUROPEEN

(11) **EP 1 064 915 A1**
(43) Date de publication de la demande: **03.01.2001**
(21) Numéro de dépôt: 00401424.7
(22) Date de dépôt: 23.05.2000
(51) Int. Cl.: A61K 7/06

(54) **Compositions cosmétiques contenant un polymère amphotère et un agent conditionneur et leurs utilisations**

(30) Priorité: 25.06.1999 FR 9908169
(71) Demandeur: L'OREAL, 75008 Paris (FR)
(72) Inventeur: Dubief, Claude, 78150 Le Chesnay (FR); Restle, Serge, 95390 Saint-Prix (FR)
(74) Mandataire: Dodin, Catherine

(57) **Abrégé**

L'invention concerne de nouvelles compositions cosmétiques comprenant dans un milieu cosmétiquement acceptable au moins un agent conditionneur choisi parmi les huiles de synthèse, les huiles végétales, les cires végétales et les composés de type céramide et au moins un polymère amphotère comprenant au moins un monomère de type (méth)acrylate ou (méth)acrylamide ayant au moins une chaîne grasse, ladite chaîne grasse ayant de 8 à 30 atomes de carbone.

Cette association apporte des propriétés cosmétiques (démêlage, douceur) nettement améliorées par rapport aux propriétés obtenues avec l'un ou l'autre des constituants utilisé seul.

Ces compositions sont utilisées pour le lavage et/ou le conditionnement des matières kératiniques telles que les cheveux ou la peau.

## Description

La présente invention concerne de nouvelles compositions cosmétiques comprenant dans un milieu cosmétiquement acceptable au moins un agent conditionneur choisi parmi les huiles de synthèse, les huiles végétales, les cires végétales, les composés de type céramide et au moins un polymère amphotère comprenant au moins un monomère de type (méth)acrylate ou (méth)acrylamide ayant au moins une chaîne grasse, ladite chaîne grasse ayant de 8 à 30 atomes de carbone.

Il est bien connu que des cheveux qui ont été sensibilisés (i.e. abîmés et/ou fragilisés) à des degrés divers sous l'action d'agents atmosphériques ou sous l'action de traitements mécaniques ou chimiques, tels que des colorations, des décolorations et/ou des permanentes, sont souvent difficiles à démêler et à coiffer, et manquent de douceur.

On a déjà préconisé dans les compositions pour le lavage ou le soin des matières kératiniques telles que les cheveux l'utilisation d'agents conditionneurs, notamment des polymères cationiques ou des silicones, pour faciliter le démêlage des cheveux et pour leur communiquer douceur et souplesse. Cependant, les avantages cosmétiques mentionnés ci-avant s'accompagnent malheureusement également, sur cheveux séchés, de certains effets cosmétiques jugés indésirables, à savoir un alourdissement de la coiffure (manque de légéreté du cheveu), un manque de lissage (cheveu non homogène de la racine à la pointe).
En outre, l'usage des polymères cationiques dans ce but présente divers inconvénients. En raison de leur forte affinité pour les cheveux, certains de ces polymères se déposent de façon importante lors d'utilisations répétées, et conduisent à des effets indésirables tel qu'un toucher désagréable, chargé, un raidissement des cheveux, et une adhésion inter-fibres affectant le coiffage. Ces inconvénients sont accentués dans le cas de cheveux fins, qui manquent de nervosité et de volume.

En résumé, il s'avère que les compositions cosmétiques actuelles contenant des agents conditionneurs, ne donnent pas complètement satisfaction.

La demanderesse a maintenant découvert que l'association d'un polymère amphotère particulier avec certains agents conditionneurs permet de remédier à ces inconvénients.

Ainsi, à la suite d'importantes recherches menées sur la question, il a maintenant été trouvé par la Demanderesse qu'en introduisant un polymère amphotère particulier dans les compositions en particulier capillaires à base d'agents conditionneurs particuliers, il est possible de limiter, voire supprimer, les problèmes généralement liés à l'emploi de telles compositions, à savoir en particulier l'alourdissement, le manque de lissage et de douceur, des cheveux, tout en conservant les autres propriétés cosmétiques avantageuses qui sont attachés aux compositions à base d'agents conditionneur.

Cette association apporte des propriétés cosmétiques nettement améliorées par rapport aux propriétés obtenues avec l'un ou l'autre des constituants utilisé seul.

Par ailleurs, les compositions de l'invention appliquées sur la peau notamment sous forme de bain moussant ou de gel douche, apportent une amélioration de la douceur de la peau.

Ainsi, selon la présente invention, il est maintenant proposé de nouvelles compositions cosmétiques, comprenant, dans un milieu cosmétiquement acceptable, au moins un agent conditionneur choisi parmi les huiles de synthèse, les huiles végétales, les cires végétales, les composés de type céramide et au moins un polymère amphotère comprenant au moins un monomère de type (méth)acrylate ou (méth)acrylamide ayant au moins une chaîne grasse, ladite chaîne grasse ayant de 8 à 30 atomes de carbone.

Un autre objet de l'invention concerne l'utilisation d'un polymère amphotère comprenant au moins un monomère de type (méth)acrylate ou (méth)acrylamide ayant au moins une chaîne grasse, ladite chaîne grasse ayant de 8 à 30 atomes de carbone dans, ou pour la fabrication d'une composition cosmétique comprenant un agent conditionneur choisi parmi les huiles de synthèse, les huiles végétales, les cires végétales et les composés de type céramide.

L'invention a également pour objet l'utilisation d'un polymère amphotère comprenant au moins un monomère de type (méth)acrylate ou (méth)acrylamide ayant au moins une chaîne grasse, ladite chaîne grasse ayant de 8 à 30 atomes de carbone dans une composition comprenant un agent conditionneur tel que ceux ci-dessus pour augmenter l'effet conditionneur de cet agent.

Les différents objets de l'invention vont maintenant être détaillés. L'ensemble des significations et définitions des composés utilisés dans la présente invention données ci-dessous sont valables pour l'ensemble des objets de l'invention.

Les polymères amphotères selon l'invention comprennent de préférence de 1 à 20 moles% de monomère comportant une chaîne grasse, et de préférence de 1,5 à 15 moles % par rapport au nombre total de moles de monomères.

Les polymères amphotères selon l'invention peuvent résulter de la copolymérisation
1) d'au moins un monomère de formule (Ia) ou (Ib): 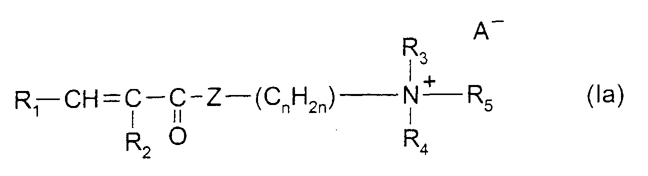 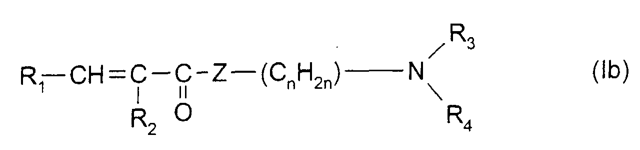 dans lesquelles R₁ et R₂, identiques ou différents, représentent un atome d'hydrogène ou un radical méthyle, R₃, R₄ et R₅, identiques ou différents, représente un radical alkyle linéaire ou ramifié ayant de 1 à 30 atomes de carbone,
   Z représente un groupe NH ou un atome d'oxygène,
   n est un nombre entier de 2 à 5,
   A⁻ est un anion issu d'un acide organique ou minéral, tel qu'un anion méthosulfate ou un halogénure tel que chlorure ou bromure.
2) d'au moins un monomère de formule (II) 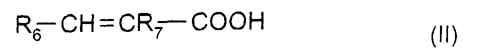 dans laquelle R₆ et R₇, identiques ou différents, représentent un atome d'hydrogène ou un radical méthyle;
   et
3) d'au moins un monomère de formule (III): 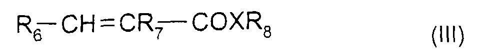 dans laquelle R₆ et R₇, identiques ou différents, représentent un atome d'hydrogène ou un radical méthyle, X désigne un atome d'oxygène ou d'azote et R₈ désigne un radical alkyle linéaire ou ramifié ayant de 1 à 30 atomes de carbone;
l'un au moins des monomères de formule (Ia), (Ib) ou (III) comportant au moins une chaîne grasse ayant de 8 à 30 atomes de carbone.

Les monomères de formule (Ia) et (Ib) de la présente invention sont choisis, de préférence, dans le groupe constitué par :
- le diméthylaminoéthylméthacrylate, le diméthylaminoéthylacrylate,
- le diéthylaminoéthylméthacrylate, le diéthylaminoéthylacrylate,
- le diméthylaminopropylméthacrylate, le diméthylaminopropylacrylate,
- le diméthylaminopropylméthacrylamide, le diméthylaminopropylacrylamide,
éventuellement quaternisés par exemple par un halogénure d'alkyle en C₁-C₄ ou un sulfate de dialkyle en C₁-C₄.

Plus particulièrement, le monomère de formule (la) est choisi parmi le chlorure d'acrylamidopropyl triméthyl ammonium et le chlorure de méthacrylamidopropyl triméthyl ammonium.

Les monomères de formule (II) de la présente invention sont choisis, de préférence, dans le groupe constitué par l'acide acrylique, l'acide méthacrylique, l'acide crotonique et l'acide méthyl-2 crotonique.
Plus particulièrement, le monomère de formule (Il) est l'acide acrylique.

Les monomères de formule (III) de la présente invention sont choisis, de préférence, dans le groupe constitué des acrylates ou méthacrylate d'alkyle en C₁₂-C₂₂ et plus particulièrement en C₁₆-C₁₈.

Les monomères constituant les polymères amphotères de l'invention sont de préférence déjà neutralisés et/ou quaternisés.

Le rapport du nombre de charges cationiques/charges anioniques est de préférence égal à environ 1.

Les polymères amphotères selon l'invention comprennent de préférence de 1 à 10 moles% du monomère comportant une chaîne grasse (monomère de formule (Ia), (Ib) ou (III)), et de préférence de 1,5 à 6 moles % .

Les poids moléculaires moyen en poids des polymères amphotères selon l'invention peuvent varier de 500 à 50.000.000 et sont de préférence compris entre 10.000 et 5 000 000.

Les polymères selon l'invention peuvent également contenir d'autre monomères tels que des monomères non ioniques et en particulier tels que les acrylates ou méthacrylates d'alkyle en C₁-C₄.

Les polymères amphotères selon l'invention sont par exemple décrits dans la demande de brevet WO9844012.

Les polymères amphotères particulièrement préférés selon l'invention sont choisis parmi les copolymères acide acrylique/chlorure d'acrylamidopropyl triméthyl ammonium/ méthacrylate de stéaryle.

Le polymère amphotère est utilisé généralement dans les compositions selon l'invention en une quantité comprise entre 0,05 et 10% en poids par rapport au poids total de la composition. De préférence, cette quantité est comprise entre 0,1 et 5% en poids par rapport au poids total de la composition.

Les agents de conditionnement peuvent se présenter sous forme liquide, semi-solide ou solide tels que par exemple des huiles, des cires ou des gommes.

Selon l'invention, les agents conditionneurs peuvent être choisis parmi les huiles de synthèse, les huiles végétales, les cires végétales, les composés de type céramide et leurs mélanges. Les agents conditionneurs préférés selon l'invention sont les poly-oléfines et les huiles végétales.

Les huiles de synthèse sont notamment les polyoléfines.
Les polyoléfines sont de préférence des poly-α-oléfines et en particulier ;
- de type polybutène, hydrogéné ou non, et de préférence polyisobutène, hydrogéné ou non.
On utilise de préférence les oligomères d'isobutylène de poids moléculaire inférieur à 1000 et leurs mélange avec des polyisobutylènes de poids moléculaire supérieur à 1000 et de préférence compris entre 1000 et 15000.
A titre d'exemples de poly-α-oléfines utilisables dans le cadre de la présente invention, on peut plus particulièrement mentionner les polyisobutènes vendus sous le nom de PERMETHYL 99 A, 101 A , 102 A , 104 A (n=16) et 106 A (n=38) par la Société PRESPERSE Inc, ou bien encore les produits vendus sous le nom de ARLAMOL HD (n=3) par la Société ICI (n désignant le degré de polymérisation),
- de type polydécène, hydrogéné ou non.
De tels produits sont vendus par exemple sous les dénominations ETHYLFLO par la société ETHYL CORP., et d'ARLAMOL PAO par la société ICI.

Selon la présente invention, les composés de type céramide sont notamment les céramides et/ou les glycocéramides et/ou les pseudocéramides et/ou les néocéramides, naturelles ou synthétiques.

Des composés de type céramide sont par exemple décrits dans les demandes de brevet DE4424530, DE4424533, DE4402929, DE4420736, WO95/23807, WO94/07844, EP-A-0646572, WO95/16665, FR-2 673 179, EP-A-0227994 et WO 94/07844, WO94/24097, WO94/10131 dont les enseignements sont ici inclus à titre de référence.

Des composés de type céramides particulièrement préférés selon l'invention sont par exemple :
- le 2-N-linoléoylamino-octadécane-1,3-diol,
- le 2-N-oléoylamino-octadécane-1,3-diol,
- le 2-N-palmitoylamino-octadécane-1,3-diol,
- le 2-N-stéaroylamino-octadécane-1,3-diol,
- le 2-N-béhénoylamino-octadécane-1,3-diol,
- le 2-N-[2-hydroxy-palmitoyl]-amino-octadécane-1,3-diol,
- le 2-N-stéaroyl amino-octadécane-1,3,4 triol et en particulier la N-stéaroyl phytos-phingosine,
- le 2-N-palmitoylamino-hexadécane-1,3-diol
- le (bis-(N-hydroxyéthyl N-cétyl) malonamide),
- le N-(2-hydroxyéthyl)-N-(3-cétyloxy-2-hydroxypropyl)amide d'acide cétylique .
- le N-docosanoyl N-méthyl-D-glucamine
ou les mélanges de ces composés.

Les huiles végétales sont choisies préférentiellement dans le groupe formé par les huiles de tournesol, de maïs, de soja, d'avocat, de jojoba, de courge, de pépins de raisin, de sésame, de noisette, l'huile de Purcellin, de palme, de ricin, de noix, de noix de cajou ou d'amande douce;
On peut également utiliser les huiles essentielles naturelles ou synthétiques telles que, par exemple, les huiles d'eucalyptus, de lavandin, de lavande, de vétivier, de litsea cubeba, de citron, de santal, de romarin, de camomille, de sarriette, de noix de muscade, de cannelle, d'hysope, de carvi, d'orange, de géraniol, de cade et de bergamote;

Les cires végétales sont des substances naturelles ou modifiées solides à température ambiante (20°-25°C). Elles sont insolubles dans l'eau, solubles dans les huiles et sont capables de former un film hydrofuge.

La cire ou les cires végétales sont choisies notamment, parmi la cire de Carnauba, la cire de Candelila, la cire d'olivier, de tournesol, la cire de riz, la cire de jojoba hydrogénée ou les cires absolues de fleurs telles que la cire essentielle de fleur de cassis vendue par la Société BERTIN (France).

Il est bien entendu possible de mettre en oeuvre des mélanges d'agents conditionneur.

Selon l'invention, le ou les agents conditionneurs peuvent représenter de 0,001 % à 20 % en poids, de préférence de 0,01 % à 10% en poids et plus particulièrement de 0,1 à 3% en poids par rapport au poids total de la composition finale.

Les compositions de l'invention contiennent en outre avantageusement au moins un agent tensioactif qui est généralement présent en une quantité comprise entre 0,1% et 60% en poids environ, de préférence entre 3% et 40% et encore plus préférentiellement entre 5% et 30%, par rapport au poids total de la composition.

Cet agent tensioactif peut être choisi parmi les agents tensioactifs anioniques, amphotères, non-ioniques, ou leurs mélanges.

Les tensioactifs convenant à la mise en oeuvre de la présente invention sont notamment les suivants :

### (i) Tensioactif(s) anionique(s) :

Leur nature ne revêt pas, dans le cadre de la présente invention, de caractère véritablement critique.
Ainsi, à titre d'exemple de tensioactifs anioniques utilisables, seuls ou mélanges, dans le cadre de la présente invention, on peut citer notamment (liste non limitative) les sels (en particulier sels alcalins, notamment de sodium, sels d'ammonium, sels d'amines, sels d'aminoalcools ou sels de magnésium) des composés suivants : les alkylsulfates, les alkyléthersulfates, alkylamidoéthersulfates, alkylarylpolyéthersulfates, monoglycérides sulfates ; les alkylsulfonates, alkylphosphates, alkylamidesulfonates, alkylarylsulfonates, α-oléfine-sulfonates, paraffine-sulfonates ; les alkylsulfosuccinates, les alkyléthersulfosuccinates, les alkylamidesulfosuccinates; les alkylsulfosuccinamates ; les alkylsulfoacétates ; les alkylétherphosphates; les acylsarcosinates ; les acyliséthionates et les N-acyltaurates, le radical alkyle ou acyle de tous ces différents composés comportant de préférence de 8 à 24 atomes de carbone, et le radical aryl désignant de préférence un groupement phényle ou benzyle. Parmi les tensioactifs anioniques encore utilisables, on peut également citer les sels d'acides gras tels que les sels des acides oléique, ricinoléique, palmitique, stéarique, les acides d'huile de coprah ou d'huile de coprah hydrogénée ; les acyl-lactylates dont le radical acyle comporte 8 à 20 atomes de carbone. On peut également utiliser des tensioactifs faiblement anioniques, comme les acides d'alkyl D galactoside uroniques et leurs sels ainsi que les acides alkyl (C₆-C₂₄) éther carboxyliques polyoxyalkylénés, les acides alkyl(C₆-C₂₄)aryl éther carboxyliques polyoxyalkylénés ,les acides alkyl(C₆-C₂₄) amido éther carboxyliques polyoxyalkylénés et leurs sels, en particulier ceux comportant de 2 à 50 groupements oxyde d'éthylène, et leurs mélanges. Parmi les tensioactifs anioniques, on préfère utiliser selon l'invention les sels d'alkylsulfates et d'alkyléthersufates et leurs mélanges.

### (ii) Tensioactif(s) non ionique(s) :

Les agents tensioactifs non-ioniques sont, eux aussi, des composés bien connus en soi (voir notamment à cet égard "Handbook of Surfactants" par M.R. PORTER, éditions Blackie & Son (Glasgow and London), 1991, pp 116-178) et leur nature ne revêt pas, dans le cadre de la présente invention, de caractère critique. Ainsi, ils peuvent être notamment choisis parmi (liste non limitative) les alcools, les alpha-diols, les alkylphénols ou les acides gras polyéthoxylés, polypropoxylés ou polyglycérolés, ayant une chaîne grasse comportant par exemple 8 à 18 atomes de carbone, le nombre de groupements oxyde d'éthylène ou oxyde de propylène pouvant aller notamment de 2 à 50 et le nombre de groupements glycérol pouvant aller notamment de 2 à 30. On peut également citer les copolymères d'oxyde d'éthylène et de propylène, les condensats d'oxyde d'éthylène et de propylène sur des alcools gras ; les amides gras polyéthoxylés ayant de préférence de 2 à 30 moles d'oxyde d'éthylène, les amides gras polyglycérolés comportant en moyenne 1 à 5 groupements glycérol et en particulier 1,5 à 4 ; les amines grasses polyéthoxylées ayant de préférence 2 à 30 moles d'oxyde d'éthylène ; les esters d'acides gras du sorbitan oxyéthylénés ayant de 2 à 30 moles d'oxyde d'éthylène ; les esters d'acides gras du sucrose, les esters d'acides gras du polyéthylèneglycol, les alkylpolyglycosides, les dérivés de N-alkyl glucamine, les oxydes d'amines tels que les oxydes d'alkyl (C₁₀ - C₁₄) amines ou les oxydes de N-acylaminopropylmorpholine. On notera que les alkylpolyglycosides constituent des tensioactifs non-ioniques rentrant particulièrement bien dans le cadre de la présente invention.

### (iii) Tensioactif(s) amphotère(s):

Les agents tensioactifs amphotères, dont la nature ne revêt pas dans le cadre de la présente invention de caractère critique, peuvent être notamment (liste non limitative) des dérivés d'amines secondaires ou tertiaires aliphatiques, dans lesquels le radical aliphatique est une chaîne linéaire ou ramifiée comportant 8 à 22 atomes de carbone et contenant au moins un groupe anionique hydrosolubilisant (par exemple carboxylate, sulfonate, sulfate, phosphate ou phosphonate) ; on peut citer encore les alkyl (C₈-C₂₀) bétaïnes, les sulfobétaïnes, les alkyl (C₈-C₂₀) amidoalkyl (C₁-C₆) bétaïnes ou les alkyl (C₈-C₂₀) amidoalkyl (C₁-C₆) sulfobétaïnes.

Parmi les dérivés d'amines, on peut citer les produits commercialisés sous les dénomination MIRANOL, tels que décrits dans les brevets US-2 528 378 et US-2 781 354 et de structures :

R₂-CONHCH₂CH₂-N(R₃)(R₄)(CH₂COO-) (2)

dans laquelle : R₂ désigne un radical alkyle dérivé d'un acide R₂-COOH présent dans l'huile de coprah hydrolysée, un radical heptyle, nonyle ou undécyle, R₃ désigne un groupement bêta-hydroxyéthyle et R₄ un groupement carboxyméthyle ;
et

R₅-CONHCH₂CH₂-N(B)(C) (3)

dans laquelle :
B représente -CH₂CH₂OX', C représente -(CH₂)_{z}-Y', avec z = 1 ou 2,
X' désigne le groupement -CH₂CH₂-COOH ou un atome d'hydrogène
Y' désigne -COOH ou le radical -CH₂ - CHOH - SO3H
R₅ désigne un radical alkyle d'un acide R₉ -COOH présent dans l'huile de coprah ou dans l'huile de lin hydrolysée, un radical alkyle, notamment en C₇, C₉, C₁₁ ou C₁₃, un radical alkyle en C₁₇ et sa forme iso, un radical C₁₇ insaturé.

Ces composés sont classés dans le dictionnaire CTFA, 5ème édition, 1993, sous les dénominations Disodium Cocoamphodiacetate, Disodium Lauroamphodiacetate, Disodium Caprylamphodiacetate, Disodium Capryloamphodiacetate, Disodium Cocoamphodipropionate, Disodium Lauroamphodipropionate, Disodium Caprylamphodipropionate, Disodium Capryloamphodipropionate, Lauroamphodipropionic acid, Cocoamphodipropionic acid.
A titre d'exemple on peut citer le cocoamphodiacetate commercialisé sous la dénomination commerciale MIRANOL C2M concentré par la société RHONE POULENC.

Dans les compositions conformes à l'invention, on utilise de préférence des mélanges d'agents tensioactifs et en particulier des mélanges d'agents tensioactifs anioniques et des mélanges d'agents tensioactifs anioniques et d'agents tensioactifs amphotères ou non ioniques. Un mélange particulièrement préféré est un mélange constitué d'au moins un agent tensioactif anionique et d'au moins un agent tensioactif amphotère.

On utilise de préférence un agent tensioactif anionique choisi parmi les alkyl(C₁₂-C₁₄) sulfates de sodium, de triéthanolamine ou d'ammonium, les alkyl (C₁₂-C₁₄)éthersulfates de sodium, de triéthanolamine ou d'ammonium oxyéthylénés à 2,2 moles d'oxyde d'éthylène, le cocoyl iséthionate de sodium et l'alphaoléfine(C₁₄-C₁₆) sulfonate de sodium et leurs mélange avec :
- soit un agent tensioactif amphotère tel que les dérivés d'amine dénommés disodiumcocoamphodipropionate ou sodiumcocoamphopropionate commercialisés notamment par la société RHONE POULENC sous la dénomination commerciale "MIRANOL C2M CONC" en solution aqueuse à 38 % de matière active ou sous la dénomination MIRANOL C32;
- soit un agent tensioactif amphotère de type zwittérionique tel que les alkylbétaïnes en particulier la cocobétaïne commercialisée sous la dénomination "DEHYTON AB 30" en solution aqueuse à 32 % de MA par la société HENKEL.

La composition de l'invention peut également contenir au moins un additif choisi parmi les épaississants, les parfums, les agents nacrants, les conservateurs, les filtres solaires siliconés ou non, les vitamines, les cires minérales, les polymères cationiques, anioniques ou non ioniques, les protéines, les hydrolysats de protéines, les acides gras à chaînes linéaires ou ramifiées en C₁₆-C₄₀ tels que l'acide méthyl-18 eicosanoique, les esters d'acide gras, les alcools gras, les hydroxyacides, les vitamines, le panthénol, les huiles animales, les huiles minérales, les tensioactifs cationiques et tout autre additif classiquement utilisé dans le domaine cosmétique qui n'affecte pas les propriétés des compositions selon l'invention.

Ces additifs sont présents dans la composition selon l'invention dans des proportions pouvant aller de 0 à 20% en poids par rapport au poids total de la composition. La quantité précise de chaque additif est déterminée facilement par l'homme du métier selon sa nature et sa fonction.

Les compositions conformes à l'invention peuvent être plus particulièrement utilisées pour le lavage ou le traitement des matières kératiniques telles que les cheveux, la peau, les cils, les sourcils, les ongles, les lèvres, le cuir chevelu et plus particulièrement les cheveux.

En particulier, les compositions selon l'invention sont des compositions détergentes telles que des shampooings, des gels-douche et des bains moussants. Dans ce mode de réalisation de l'invention, les compositions comprennent une base lavante, généralement aqueuse.

Le ou les tensioactifs formant la base lavante peuvent être indifféremment choisis, seuls ou en mélanges, au sein des tensioactifs anioniques, amphotères, non ioniques et cationiques tels que définis ci-dessus.

La quantité et la qualité de la base lavante sont celles suffisantes pour conférer à la composition finale un pouvoir moussant et/ou détergent satisfaisant.

Ainsi, selon l'invention, la base lavante peut représenter de 4 % à 50 % en poids, de préférence de 6 % à 35 % en poids, et encore plus préférentiellement de 8 % à 25 % en poids, du poids total de la composition finale.

L'invention a encore pour objet un procédé de traitement des matières kératiniques telles que la peau ou les cheveux, caractérisé en ce qu'il consiste à appliquer sur les matières kératiniques une composition cosmétique telle que définie précédemment, puis à effectuer éventuellement un rinçage à l'eau.

Ainsi, ce procédé selon l'invention permet le maintien de la coiffure, le traitement, le soin ou le lavage ou le démaquillage de la peau, des cheveux ou de toute autre matière kératinique.

Les compositions de l'invention peuvent également se présenter sous forme d'après-shampooing à rincer ou non, de compositions pour permanente, défrisage, coloration ou décoloration des cheveux, ou encore sous forme de compositions à rincer, à appliquer avant ou après une coloration, une décoloration, une permanente ou un défrisage ou encore entre les deux étapes d'une permanente ou d'un défrisage.

Les compositions de l'invention peuvent encore se présenter sous la forme de compositions pour la peau, et en particulier sous la forme de solutions ou de gels pour le bain ou la douche ou de produits démaquillants.

Les compositions selon l'invention peuvent également se présenter sous forme de lotions aqueuses ou hydroalcooliques pour le soin de la peau et/ou des cheveux.

Les compositions cosmétiques selon l'invention peuvent se présenter sous forme de gel, de lait, de crème, d'émulsion, de lotion épaissie ou de mousse et être utilisées pour la peau, les ongles, les cils, les lèvres et plus particulièrement les cheveux.

Les compositions peuvent être conditionnées sous diverses formes notamment dans des vaporisateurs, des flacons pompe ou dans des récipients aérosols afin d'assurer une application de la composition sous forme vaporisée ou sous forme de mousse. De telles formes de conditionnement sont indiquées, par exemple, lorsqu'on souhaite obtenir un spray, une laque ou une mousse pour le traitement des cheveux.

Dans tout ce qui suit ou ce qui précède, les pourcentages exprimés sont en poids.

L'invention va être maintenant plus complètement illustrée à l'aide des exemples suivants qui ne sauraient être considérés comme la limitant aux modes de réalisation décrits. Dans les exemples, MA signifie matière active.

### EXEMPLE 1

On a préparé le shampooing de composition suivante :

| | |
|---|---|
| Lauryléther sulfate de sodium (2,2 OE) | 15g MA |
| Cocoyl bétaïne en solution aqueuse à 30% (DEHYTON AB 30 de HENKEL) | 5g MA |
| 2,2,4,4,6,6,8-heptamethylnonane (ISOHEXADECANE de BAYER) | 1.5g MA |
| Terpolymère de chlorure de méthacrylamidopropyl triméthyl ammonium, d'acide acrylique et de méthacrylate de stéaryle (49% / 49% / 2% molaire) | 1g MA |
| Chlorure de Sodium | 4g MA |
| Conservateur | q. s |
| pH ajusté à 7 | |
| Eau q. s. p | 100 g |

Les cheveux traités avec ce shampooing sont doux, lisses et se démêlent facilement.

### EXEMPLE 2

On a préparé le shampooing de composition suivante :

| | |
|---|---|
| Lauryléther sulfate de sodium (2,2 OE) | 9.8g MA |
| Cocoyl amidopropyl bétaïne en solution aqueuse à 38 % de MA (TEGO BETAINE F 50 de GOLDSCHMIDT ) | 1.6g MA |
| Acide lauryl éther carboxylique (4.5 OE) (AKYPO RLM 45 CA de CHEM Y ) | 5.6g MA |
| Huile d'avocat (LIPOVOL A STABILISEE de LIPO CHEMICALS) | 3 g MA |
| Terpolymère de chlorure de méthacrylamidopropyltriméthyl ammonium, d'acide acrylique et de méthacrylate de stéaryle (49% / 49% / 2% molaire) | 0.2 g MA |
| Acide polyacrylique réticulé (CARBOPOL 980 de GOODRICH) | 0.25 g MA |
| Distéarate de glycol (EMPILAN D 2039 de ALBRIGHT & WILSON) | 2 g MA |
| Chlorure de Sodium | 1 g MA |
| Conservateur | q. s |
| pH ajusté à 7 | 7 |
| Eau q. s. p | 100 g |

Les cheveux traités avec ce shampooing sont doux, lisses et se démêlent facilement.

### EXEMPLE 3

On a préparé une composition d'après-shampooing à rincer de composition suivante :

| | |
|---|---|
| Chlorure de béhényl triméthyl ammonium a 80 % dans un mélange eau/isopropanol (15/85) (GENAMIN KDMP de CLARIANT) | 2 g MA |
| Mélange alcool cétylstéarylique / alcool cétylstéarylique oxyéthyléné (30 OE) (EMPIWAX CL de ALBRIGHT & WILSON) | 4 g MA |
| Méthosulfate de méthyl suifamidoéthyl imidazolinium à 75 % de MA dans le propylène glycol (REWOQUAT W 75 PG de WITCO) | 0.05 g MA |
| Terpolymère de chlorure de méthacrylamidopropyltriméthyl ammonium, d'acide acrylique et de méthacrylate de stéaryle (49% / 49% / 2% molaire) | 0.75 g MA |
| N-oléoyl dihydrosphingosine | 0.01 g MA |
| | |
| Conservateur | q. s |
| pH ajusté à 7 | 7 |
| Eau q. s. p | 100 g |

Les cheveux traités avec cet après-shampooing sont doux, lisses et se démêlent facilement.

## Revendications

1. Composition cosmétique, caractérisée par le fait qu'elle comprend, dans un milieu cosmétiquement acceptable, au moins au moins un agent conditionneur choisi parmi les huiles de synthèse, les huiles végétales, les cires végétales, les composés de type céramide et au moins un polymère amphotère comprenant au moins un monomère de type (méth)acrylate ou (méth)acrylamide ayant au moins une chaîne grasse, ladite chaîne grasse ayant de 8 à 30 atomes de carbone.

2. Composition selon la revendication 1, caractérisée par le fait que ledit polymère amphotères comprend de 1 à 20 moles% de monomère comportant une chaîne grasse, et de préférence de 1,5 à 15 moles % par rapport au nombre total de moles de monomères.

3. Composition selon l'une quelconque des revendications 1 ou 2, caractérisée par le fait les polymères amphotères résultent de la copolymérisation
1) d'au moins un monomère de formule (Ia) ou (Ib): 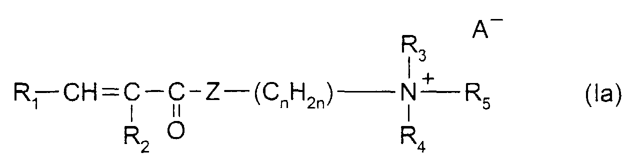 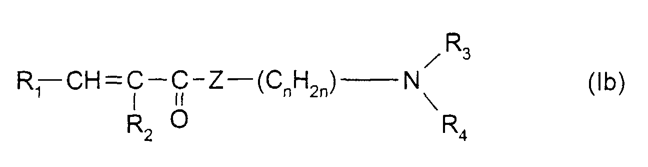 dans lesquelles R₁ et R₂, identiques ou différents, représentent un atome d'hydrogène ou un radical méthyle, R₃, R₄ et R₅, identiques ou différents, représente un radical alkyle linéaire ou ramifié ayant de 1 à 30 atomes de carbone,
Z représente un groupe NH ou un atome d'oxygène,
n est un nombre entier de 2 à 5,
A⁻ est un anion issu d'un acide organique ou minéral.
2) d'au moins un monomère de formule (II) 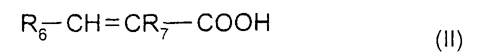 dans laquelle R₆ et R₇, identiques ou différents, représentent un atome d'hydrogène ou un radical méthyle;
et
3) d'au moins un monomère de formule (III): 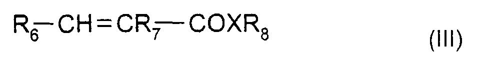 dans laquelle R₆ et R₇, identiques ou différents, représentent un atome d'hydrogène ou un radical méthyle, X désigne un atome d'oxygène ou d'azote et R₈ désigne un radical alkyle linéaire ou ramifié ayant de 1 à 30 atomes de carbone;
l'un au moins des monomères de formule (Ia), (Ib) ou (III) comportant au moins une chaîne grasse ayant de 8 à 30 atomes de carbone.

4. Composition selon la revendications 3, caractérisée par le fait que les monomères de formule (Ia) et (Ib) de la présente invention sont choisis dans le groupe constitué par :
- le diméthylaminoéthylméthacrylate, le diméthylaminoéthylacrylate,
- le diéthylaminoéthylméthacrylate, le diéthylaminoéthylacrylate,
- le diméthylaminopropylméthacrylate, le diméthylaminopropylacrylate,
- le diméthylaminopropylméthacrylamide, le diméthylaminopropylacrylamide, éventuellement quaternisés

5. Composition selon l'une quelconque des revendications 3 ou 4, caractérisée par le fait que le monomère de formule (la) est choisi parmi le chlorure d'acrylamidopropyl triméthyl ammonium et le chlorure de méthacrylamidopropyl triméthyl ammonium.

6. Composition selon l'une quelconque des revendications 3 à 5, caractérisée par le fait que les monomères de formule (II) sont choisis dans le groupe constitué par l'acide acrylique, l'acide méthacrylique, l'acide crotonique et l'acide méthyl-2 crotonique.

7. Composition selon l'une quelconque des revendications 3 à 6, caractérisée par le fait que les monomères de formule (III) sont choisis dans le groupe constitué des acrylates ou méthacrylate d'alkyle en C₁₂-C₂₂ et plus particulièrement en C₁₆-C₁₈.

8. Composition selon l'une quelconque des revendications 1 à 7, caractérisée par le fait que lesdits polymères amphotères sont choisis parmi les copolymères acide acrylique/chlorure d'acrylamidopropyl triméthyl ammonium/ méthacrylate de stéaryle.

9. Composition selon l'une quelconque des revendications précédentes, caractérisée par le fait que ledit polymère amphotère est utilisé dans les compositions selon l'invention en une quantité comprise entre 0,05 et 10% en poids par rapport au poids total de la composition.

10. Composition selon l'une quelconque des revendications précédentes, caractérisée par le fait que les huiles de synthèse sont des polyoléfines.

11. Composition selon la revendication 10, caractérisée par le fait que les polyoléfines sont de type polybutène, hydrogéné ou non, ou de type polydécène, hydrogéné ou non.

12. Composition selon l'une quelconque des revendications précédentes, caractérisée par le fait que les composés de type céramide sont choisis parmi :
- le 2-N-linoléoylamino-octadécane-1,3-diol,
- le 2-N-oléoylamino-octadécane-1,3-diol,
- le 2-N-palmitoylamino-octadécane-1,3-diol,
- le 2-N-stéaroylamino-octadécane-1,3-diol,
- le 2-N-béhénoylamino-octadécane-1,3-diol,
- le 2-N-[2-hydroxy-palmitoyl]-amino-octadécane-1,3-diol,
- le 2-N-stéaroyl amino-octadécane-1,3,4 triol et en particulier la N-stéaroyl phytos-phingosine,
- le 2-N-palmitoylamino-hexadécane-1,3-diol
- le (bis-(N-hydroxyéthyl N-cétyl) malonamide),
- le N-(2-hydroxyéthyl)-N-(3-cétyloxy-2-hydroxypropyl)amide d'acide cétylique .
- le N-docosanoyl N-méthyl-D-glucamine ou les mélanges de ces composés.

13. Composition selon l'une quelconque des revendications précédentes, caractérisée par le fait que les huiles végétales sont choisies dans le groupe formé par les huiles de tournesol, de maïs, de soja, d'avocat, de jojoba, de courge, de pépins de raisin, de sésame, de noisette, l'huile de Purcellin, de palme, de ricin, de noix, de noix de cajou ou d'amande douce, les huiles essentielles naturelles ou synthétiques telles que, par exemple, les huiles d'eucalyptus, de lavandin, de lavande, de vétivier, de litsea cubeba, de citron, de santal, de romarin, de camomille, de sarriette, de noix de muscade, de cannelle, d'hysope, de carvi, d'orange, de géraniol, de cade et de bergamote.

14. Composition selon l'une quelconque des revendications précédentes, caractérisée par le fait les cires végétales sont choisies parmi la cire de Carnauba, la cire de Candelila, la cire d'olivier, de tournesol, la cire de riz, la cire de jojoba hydrogénée et les cires absolues de fleurs telles que la cire essentielle de fleur de cassis.

15. Composition selon l'une quelconque des revendications précédentes, caractérisée en ce que l'agent conditionneur est présent à une concentration comprise entre 0,001 % et 20 % en poids par rapport au poids total de la composition, de préférence entre 0,01 % et 10 % en poids.

16. Composition selon l'une quelconque des revendications précédentes, caractérisée par le fait qu'elle comprend en outre au moins un agent tensioactif.

17. Composition selon la revendication 16, caractérisée par le fait que le ou les agents tensioactifs sont présents à une concentration comprise entre 0,1% et 60% en poids, de préférence entre 3% et 40% en poids, par rapport au poids total de la composition.

18. Composition selon l'une quelconque des revendications précédentes, caractérisée par le fait qu'elle comprend en outre au moins un additif choisi parmi les épaississants, les parfums, les agents nacrants, les conservateurs, les filtres solaires siliconés ou non, les vitamines, les cires minérales, les polymères cationiques, anioniques ou non ioniques, les protéines, les hydrolysats de protéines, les acides gras à chaînes linéaires ou ramifiées en C₁₆-C₄₀ tels que l'acide méthyl-18 eicosanoique, les esters d'acide gras, les alcools gras, les hydroxyacides, les vitamines, le panthénol, les huiles animales, les huiles minérales, les tensioactifs cationiques et tout autre additif classiquement utilisé dans le domaine cosmétique.

19. Composition selon l'une quelconque des revendications précédentes, caractérisée par le fait qu'elle se présente sous forme de shampooing, d'après-shampooing, de composition pour la permanente, le défrisage, la coloration ou la décoloration des cheveux, de composition à rincer à appliquer entre les deux étapes d'une permanente ou d'un défrisage, de composition pour la peau.

20. Utilisation d'une composition telle que définie dans l'une quelconque des revendications précédentes pour le lavage ou pour le soin des matières kératiniques.

21. Procédé de traitement des matières kératiniques, telles que les cheveux, caractérisé en ce qu'il consiste à appliquer sur lesdites matières une composition cosmétique selon l'une des revendications 1 à 19, puis à effectuer éventuellement un rinçage à l'eau.

22. Utilisation d'un polymère amphotère comprenant au moins un monomère de type (méth)acrylate ou (méth)acrylamide ayant au moins une chaîne grasse, ladite chaîne grasse ayant de 8 à 30 atomes de carbone dans une composition comprenant un agent conditionneur choisi parmi les huiles de synthèse, les huiles végétales, les cires végétales et les composés de type céramide pour augmenter l'effet conditionneur de cet agent.

23. Utilisation d'un polymère amphotère à chaîne grasse tel que défini à l'une des revendications 1 à 8 dans, ou pour la fabrication d'une composition cosmétique comprenant un agent conditionneur choisi parmi les huiles de synthèse, les huiles végétales, les cires végétales et les composés de type céramide.
